# EUROPEAN PATENT SPECIFICATION

(11) **EP 4 313 059 B1**
(45) Date of publication and mention of the grant of the patent: **30.09.2026**
(21) Application number: 22719273.9
(22) Date of filing: 29.03.2022
(51) Int. Cl.: A61K 31/5377, A61P 29/00, A61P 35/00, A61P 35/02, A61P 35/04, A61K 9/20, A61K 31/337, A61K 31/437, A61K 31/7068, A61K 39/395, A61K 45/06

(54) **ROGINOLISIB FOR THE TREATMENT OF PANCREATIC CANCER**
ROGINOLISIB ZUR BEHANDLUNG VON PANKREASKREBS
ROGNINOLISIB POUR LE TRAITEMENT DU CANCER DU PANCRÉAS

(30) Priority: 29.03.2021 GB 202104416; 09.06.2021 GB 202108242; 03.12.2021 GB 202117511
(43) Date of publication of application: 07.02.2024
(73) Proprietor: iOnctura SA, 1202 Genève (CH)
(72) Inventor: JOHNSON, Zoë, 1202 Genève (CH); NIEWOLA-STASZKOWSKA, Karolina, 1202 Genève (CH); VAN DER VEEN, Lars, 1202 Genève (CH); KONSTANTINIDOU, Georgia, 3010 Bern (CH)
(74) Representative: Mewburn Ellis LLP
(86) International application number: PCT/EP2022/058296
(87) International publication number: WO 2022/207648

(56) References cited:
- WO-A1-2011/058149
- WO-A1-2014/121901
- WO-A1-2016/124939
- MACQUEEN AMY R. ET AL: "Abstract 666: A novel, highly selective PI3K[delta] inhibitor for the treatment of solid malignancies that express high levels of target protein as assessed by immunohistochemistry", CANCER RESEARCH, vol. 80, no. 16_Supplement, 15 August 2020 (2020-08-15), US, pages 666 - 666, XP055941409, ISSN: 0008-5472, DOI: 10.1158/1538-7445.AM2020-666
- PAPAKONSTANTI E ET AL: "Preclinical development of a novel, highly selective PI3K[delta] inhibitor, IOA-244 for the treatment of solid malignancies", 7 November 2019 (2019-11-07), London, UK, pages 1, XP055941402, Retrieved from the Internet <URL:https://www.ionctura.com/publications/MAP_2019_poster.pdf> [retrieved on 20220712]
- JOHNSON Z. ET AL: "Preclinical development of a novel, highly selective PI3Kdelta inhibitor, IOA- 244, for the treatment of solid malignancies", 9 November 2019 (2019-11-09), XP055941748, Retrieved from the Internet <URL:https://www.sciencedirect.com/science/article/pii/S0923753419619915?via%3Dihub> [retrieved on 20220712], DOI: 10.1093/annonc/mdz413
- CONWAY JAMES RW ET AL: "Combating pancreatic cancer with PI3K pathway inhibitors in the era of personalised medicine", GUT MICROBIOTA, vol. 68, no. 4, 5 November 2018 (2018-11-05), UK, pages 742 - 758, XP055942939, ISSN: 0017-5749, DOI: 10.1136/gutjnl-2018-316822

## Description

This application claims priority from GB2104416.9 filed 29 March 2021, from GB2108242.5 filed 9 June 2021, and from GB 2117511.2 filed 3 December 2021.

### Field of the Invention

The present invention relates to a compound or pharmaceutically acceptable salt thereof for use in a method of treatment of pancreatic cancer, and to combination methods including said compound.

### Background

Pancreatic cancer is a malignant tumour of the pancreas. Pancreatic cancer has been called a 'silent' disease because early pancreatic cancer usually does not cause symptoms. It is therefore difficult to detect in its early stages.

Pancreatic cancer is one of the deadliest types of cancer with a very poor 5-year survival rate of only 7%. A mere 25% of pancreatic cancer patients are surgical candidates at the time of diagnosis, and of those who receive surgical resection, only about 20% live longer than 5 years. Chemotherapy with gemcitabine is a standard treatment with a 5-10% response rate and average median overall survival of 6 months (Burris et al. 1997).

Pancreatic ductal adenocarcinoma (PDAC), the most prevalent form of pancreatic cancer, is a growing health problem with increasing mortality worldwide, exerting a huge economic burden on our healthcare system and significantly impacting the quality of life of patients. It is predicted that PDAC will become the second leading cause of cancer death in some regions. The incidence of pancreatic cancer is increasing in the Western world and a better understanding of the risk factors and symptoms associated with this disease is needed to inform both health professionals and the general population of potential preventive and/or early detection measures. There is currently a lack of therapeutic approaches for early-stage detection which would increase patient survival.

Pancreatic cancer progression typically features a dramatic desmoplastic reaction, including fibroblasts, immune cells, and a dense extracellular matrix. Because of this highly fibrotic tumour microenvironment (TME), conventional chemotherapy and radiotherapy have only moderate anti-tumour activity in pancreatic tumours. Similarly, immune therapies, which are highly effective in other cancer types, such as anti-PD-1 therapy, have shown to be ineffective in pancreatic cancer. Therefore newtreatments for pancreatic cancer, especially those that can enhance the efficacy of immune therapies, are desperately searched for.

The PI3K pathway is frequently activated in a variety of solid tumours and haematological malignancies, making PI3K an attractive therapeutic target in oncology. The pathway is also essential for many cancer-associated activities, including endothelial cell sprouting for angiogenesis, macrophage transcriptional reprogramming, T cell differentiation and homeostasis and fibroblast-supported chemoresistance (Conway et al 2019). Collectively, this suggests that application of PI3K pathway inhibitors as pancreatic cancer therapy may provide an opportunity for dual targeting of cancer cells and the deregulated cancer-associated stromal components. Conway et al. also describe the use of combination therapies to target the PI3K pathway, and discusses recent clinical trials where PDAC patients were treated with a combination of gemcitabine and nab-paclitaxel.

Pancreatic cancer is regularly associated with PI3K pathway activation and patients with high PI3K pathway activity show a significantly poorer survival than those with low activation. Given the varied roles of different PI3K isoforms in both the tumour and associated stromal cells, isoform-specific inhibitors provide isolated targeting of oncogenic signalling and in theory allow redundancy to alleviate off-target side effects in healthy tissues. However, despite promising efficacy of isoform specific inhibitors in preclinical models in monotherapy or in combination, clinical development of these inhibitors has been severely hampered by tolerability issues such as exemplified by a recent study of PI3Kδ inhibitor idelalisib in pancreatic cancer patients (Borazanci et al 2020). Hence isoform specific PI3K inhibitors for the treatment pancreatic cancer, especially those that are well tolerated, are desperately searched for.

In view of the above, there is a need in the art for PI3K modulators that inhibit specific PI3K isoforms, in particular PI3Kδ, for the treatment of pancreatic cancer. Moreover, there is a need in the art for improved ways of treating pancreatic cancer by (a combination of) compounds with high selectivity and favourable safety profiles.

The present inventors, recognising this need, set about devising the present invention.

Amongst the myriad disclosures relating to PI3K inhibitor compounds, WO2011058149 describes Tricyclic Pyrazol Amine Derivatives which are PI3K inhibitors and their use for treating autoimmune diseases, inflammatory disorders, multiple sclerosis and other diseases like cancers.

WO2014/121901 describes the hemifumarate salt of one of the PI3K inhibitor compounds of WO2011058149 which is particularly suited for the manufacture of solid oral dosage forms, such as tablets.

The use of this compound for the treatment of solid malignancies is described in Macqueen et al. (2020), Papakonstanti et al. (2019) and Johnson et al (2019).

WO2016/124939 describes various ATX inhibitor compounds and their use in the treatment of proliferative disorders in which ATX activity is implicated. Pancreatic cancer is mentioned as a possible condition that could be treated with these compounds.

### Summary of the Invention

The present invention is directed to a compound for use in the treatment of pancreatic cancer. The compound is an Pl3K-delta inhibitor. The inventors recognised that PI3Kδ inhibitors may be useful in targeted therapy for the treatment of pancreatic cancer.

In a first aspect, the invention provides a compound of Formula I: 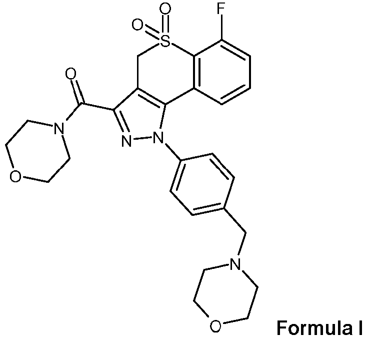 or a pharmaceutically acceptable salt thereof for use in a method of treatment of pancreatic cancer in a patient. The compound of Formula I may be referred to herein as "Compound 1".

The term pancreatic cancer includes any exocrine or neuroendocrine pancreatic cancer type. In some cases, the pancreatic cancer is pancreatic ductal adenocarcinoma (PDAC). PDAC is the most prevalent neoplastic disease of the pancreas, accounting for more than 90% of all pancreatic malignancies.

Compound 1 may be used as a monotherapy. The invention further relates to combination therapy methods.

In some cases, the treatment is a combination therapy and comprises administration of a compound of Formula II: 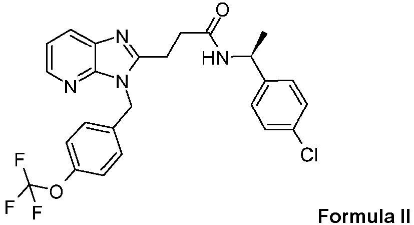 or a pharmaceutically acceptable salt thereof. The compound of Formula II may be referred to herein as "Compound 2".

The combination of Compound 1 and Compound 2 may be synergistic, and/or may otherwise result in improved treatment outcomes or patient prognosis.

Suitably, Compound 1 is administered in a pharmaceutical composition comprising Compound 1 or a pharmaceutically acceptable salt thereof and a pharmaceutically acceptable diluent, carrier or excipient. Preferably, but not necessarily, the pharmaceutical composition is suitable for oral administration. Suitably, Compound 2 is administered in a pharmaceutical composition comprising Compound 2 or a pharmaceutically acceptable salt thereof and a pharmaceutically acceptable diluent, carrier or excipient. Preferably, but not necessarily, the pharmaceutical composition is suitable for oral administration.

The treatment may comprise administration of a checkpoint inhibitor. Suitably, the checkpoint inhibitor targets PD-1, PD-L1 or LAG3. For example, the checkpoint inhibitor may be an anti-PD-1 and/or anti-PDL1 monoclonal antibody. Exemplary checkpoint inhibitors are described herein.

Accordingly, in some cases the method may comprise administration of Compound 1 or a salt thereof and a checkpoint inhibitor. In some cases, the method may comprise administration of Compound 1 or a salt thereof, Compound 2 or a salt thereof, and a checkpoint inhibitor (a triple therapy).

In some cases, the treatment is a combination therapy and comprises one or more additional chemotherapeutic agents.

For example, a method described herein may comprise administration of a therapeutically effective amount of an additional chemotherapeutic agent, optionally two additional chemotherapeutic agents. Suitable chemotherapeutics agents include gemcitabine and nab-paclitaxel. Accordingly, methods of the present invention may comprise administration of gemcitabine and/or nab-paclitaxel. Combination therapies may result in improved activity (tumour growth inhibition) and/or reduced adverse effects when compared to the chemotherapeutic agent alone.

Accordingly, in some cases the method may comprise administration of Compound 1 or a salt thereof and one or more additional chemotherapeutic agents, for example gemcitabine and/or nab-paclitaxel. In some cases, the method may comprise administration of Compound 1 or a salt thereof, a checkpoint inhibitor (for example an anti-PD-1 and/or anti-PDL1 monoclonal antibody) and one or more additional chemotherapeutic agents (for example gemcitabine and/or nab-paclitaxel).

Accordingly, in some cases the method may comprise administration of Compound 1 or a salt thereof, Compound 2 or a salt thereof, and one or more additional chemotherapeutic agents, for example gemcitabine and/or nab-paclitaxel. In some cases, the method may comprise administration of Compound 1 or a salt thereof, Compound 2 or a salt thereof, a checkpoint inhibitor (for example an anti-PD-1 and/or anti-PDL1 monoclonal antibody) and one or more additional chemotherapeutic agents (for example gemcitabine and/or nab-paclitaxel).

### Summary of the Figures

Embodiments and experiments illustrating the principles of the invention will now be discussed with reference to the accompanying figures in which:
**Figures 1a** and **b** show the activity of Compound 1 and the activity of Compound 1 in combination with an ATX inhibitor in the KPC mouse model of pancreatic cancer.
**Figures 2a** and **b** show the activity of Compound 1 in combination with a checkpoint inhibitor in the Pan02 mouse model of pancreatic cancer.
**Figure 3** shows the effect of the combination of Compound 1 with a checkpoint inhibitor on body weight in the Pan02 mouse model of pancreatic cancer.
**Figures 4a****,** **b** **and** **c** show the activity of Compound 1 and the activity of Compound 1 in combination with an ATX inhibitor in the orthotopic KPC mouse model of pancreatic cancer.

### Detailed Description of the Invention

Aspects and embodiments of the present invention will now be discussed. Further aspects and embodiments will be apparent to those skilled in the art.

### Compound 1

Compound 1 is example 339 in WO2011/058149. Its structure is according to Formula I:

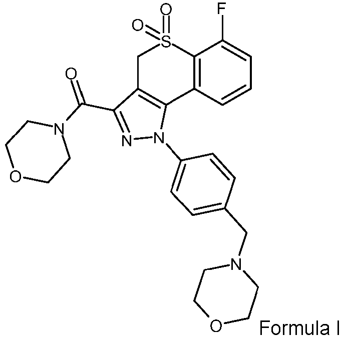

In IUPAC nomenclature, the above Compound 1 may be referred to as 6-Fluoro-3-(morpholin-4-ylcarbonyl)-1-[4-(morpholin-4-ylmethyl)phenyl]-1,4-dihydrothiochromeno[4,3-c]pyrazole 5,5-dioxide. Alternatively, the structural formula shown above may be described as [6-fluoro-1-(4-morpholin-4-yl-methylphenyl)-5,5-dioxo-4,5-dihydro-1H-5λ6-thiochromeno[4,3-C]pyrazol-3-yl]-morpholin-4-yl-methanone.

Compound 1 can be prepared and characterized as described in published patent application WO 2011/058149 A1 (see compound 339 on p. 69; the preparation on p. 303-307; and the characterization on p. 481 with p. 414-418).

Based on the process disclosed in WO 2011/058149 A1, the authors of Haselmayer et al. describe a five-step preparation procedure for the compound (Haselmayer, 2014). This procedure starts with reaction of 8-fluoro-2,3-dihydro-4Hthiochromen-4-one with diethyl oxalate in the presence of sodium ethoxide. The intermediate is cyclized with 4-(4-hydrazinylbenzyl)morpholine to form a pyrazole ring. The thioether is then oxidized to the corresponding sulfone by reaction with meta-chloroperbenzoic acid, followed by saponification of the ethyl ester into the corresponding acid and subsequent coupling with morpholine to yield the compound of formula I.

Alternatively, the intermediate of the reaction of 8-fluoro-2,3-dihydro-4H-thiochromen-4-one with diethyl oxalate in the presence of sodium ethoxide is cyclized with 4-hydrazinobenzoic acid. The benzoic acid is reduced using borane-THF complex and the thioether is oxidized to the corresponding sulfone by reaction with meta-chloroperbenzoic acid. Saponification of the ethyl ester into the corresponding acid and chlorination of both the acid and alcohol with excess 20 thionyl chloride in the presence of dimethylformamide and subsequent coupling with morpholine then yields Compound 1.

Compound 1 may be provided as a pharmaceutically acceptable salt. Suitable pharmaceutically acceptable salts are known in the art. Some pharmaceutically acceptable salts of Compound 1 are described in WO2014121901.

As used herein, Compound 1 is provided as an anhydrous hemifumarate salt (formula illustrated). Its synthesis and characterisation are described in WO2014121901 (page 4). It is referred to as solid form A1. A hemifumarate hydrate (H1) has also been identified. The anhydrous hemifumarate salt used is crystalline and has a powder X-ray peak list as described in WO2014121901. It will be appreciated that the findings of the invention are not limited to use of this solid form, although it is preferred.

Accordingly, in some cases Compound 1 is administered as the hemifumarate salt (Formula la). However, it will be understood that the invention is not so limited, and other solid forms (for example, other pharmaceutically acceptable salts) are envisaged.

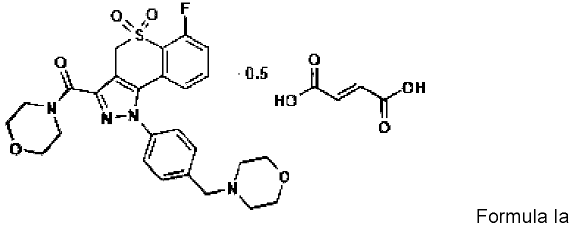

Haselmayer et al. also describe the characterisation of the compound as highly selective PI3Kδ inhibitor. The favourable *in vitro* and *in vivo* properties of Compound 1 are further described by Johnson et al. (Johnson, Z. et al., AACR 2020, poster 666).

### Compound 2

WO2016124939 describes various ATX inhibitor compounds and their use in the treatment of proliferative disorders in which ATX activity is implicated, including Compound 2.

Compound 2 is example 40 in WO2016124939. WO2016124939 describes over 200 examples. Compound 2's structure is according to Formula II.

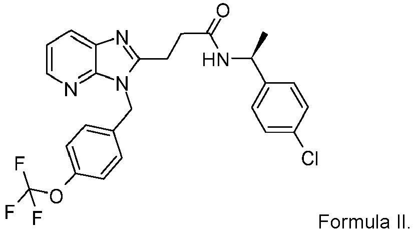

Its IUPAC name is *N*-[(*S*)-1-(4-chloro-phenyl)-ethyl]-3-[3-( 4-trifluoromethoxy-benzyl)-3H-imidazo[4,5-b]pyridin-2-yl]-propionamide. Its synthesis and characterisation are described in WO2016124939 at pages 77 and 82, respectively.

Compound 2 may be provided and administered as the free base or as a pharmaceutically acceptable salt. In some cases, Compound 2 is provided and administered as the free base.

### Checkpoint Inhibitors

Some methods of the invention are directed to combination therapy that includes checkpoint inhibitors. The potential for checkpoint inhibitors in the treatment of cancer is recognised in the art. Checkpoint inhibitor drugs may target either the PD-1 or PD-L1 protein to block PD-1 to PD-L1 binding, or target LAG3, and boost immune response against cancer cells.

In some methods of the present invention, Compound 1 is administered in combination with a PD-1 inhibitor (also referred to as anti-PD-1). Examples of anti-PD-1 monoclonal antibodies include pembrolizumab (Keytruda^{®}), nivolumab (Opdivo^{®}), cemiplimab (Libtayo^{®}), spartalizumab, camrelizumab, sintilimab, dostarlimab, tislelizumab, toripalimab and retifanlimab.

In some methods of the present invention, Compound 1 is administered in combination with a PD-L1 inhibitor (also referred to as anti-PD-L1). Examples of anti-PD-L1 monoclonal antibodies include atezolizumab (Tecentriq^{®}), durvalumab (Imfinzi^{®}), avelumab (Bavencio^{®}), M7824, and KN035.

In some methods of the present invention, Compound 1 is administered in combination with a LAG3 inhibitor (also referred to as anti-LAG3). Examples of anti-LAG3 monoclonal antibodies include relatlimab, tebotelimab, faveselimab, leramilimab, IMP-321. TSR-033, SYM-022, INCAGN02385, RG6139, and BI754111.

Checkpoint inhibitors are typically administered by I.V. infusion. Suitable doses and dosage regimens are known for each approved checkpoint inhibitor drug.

### Methods of the invention

As described in more detail below, the present inventors have surprisingly found that Compound 1 shows robust anti-tumour activity in preclinical models of pancreatic cancer and is well tolerated. Thus, a PI3K inhibitor with specificity for the δ isoform, anti-tumour activity and favourable safety characteristics in pancreatic cancer can be provided.

Additionally, the present inventors surprisingly found that the Compound 1 increases the anti-tumour activity of immune therapies such as autotaxin (ATX) and checkpoint inhibition. Thus, a specific PI3Kδ inhibitor that can increase the efficacy of immune therapy in pancreatic cancer can be provided.

In other words inventors have found that, surprisingly, pancreatic cancer can be treated with Compound 1 with or without immune therapies and this treatment is well tolerated.

Accordingly, the present invention is directed to methods of treatment of pancreatic cancer using Compound 1. Compound 1 may be suitable as a monotherapy or may be provided in combination with another agent.

In more detail, the inventors have found that Compound 1 has a favourable safety profile in humans, in particular with regard hepatotoxicity, diarrhoea/colitis, respiratory infections, and hematologic toxicities. Moreover, treatment of patients with Compound 1 does not lead to elevated liver enzymes, diarrhoea and neutropenia. Thus, a PI3K inhibitor with specificity for the isoform δ and favourable safety characteristics in patients can be provided. These results are described in GB2104416.9 (filed 29 March 2021).

Further advantageous properties of methods of treatment using Compound 1 may include one or more of higher efficacy, long treatment duration, and few dose reductions or interruptions or discontinuations.

In other words the inventors have found that, surprisingly, patients can be treated with Compound 1 with fewer adverse effects than would be expected for treatment regimens using a PI3K inhibitor. This may make treatment suitable for long term prescription without dose reduction or interruption.

The dose of Compound 1 may be provided once daily (QD) or twice daily (BID), preferably once daily, and preferably but not necessarily administered orally. Other methods of administration may be used. A suitable daily dose may be between 5 mg and 2 g, for example between 10 mg and 1g. In some cases, where Compound 1 is administered in a combination therapy, administration of Compound 1 continues during pauses in administration of other agents (for example, during days 21-28 of 28 day chemotherapeutic cycles).

In some cases Compound 1 is administered as the hemifumarate salt. However, it will be understood that the invention is not so limited, and other solid forms (for example, other pharmaceutically acceptable salts) are envisaged.

The weight equivalent of Compound 1 as a free base in a 40 mg dose is calculated at about 36 mg (that is, about 90% of the weight of Compound 1 hemifumarate corresponds to the free base, with the remaining about 10% of the weight corresponding to the salt former acid.

An exemplary method may comprise administration of Compound 1 in an amount of between 18 mg and 108 mg per day, for example between 18 mg and 72 mg per day. In some cases, the amount is between 27 mg and 90 mg per day, for example between 27 mg and 54 mg per day. In some cases, the amount is between 27 mg and 45 mg per day, for example about 36 mg per day. In some cases, the amount is between 54 mg and 90 mg per day, for example about 72 mg per day.

Where Compound 1 is administered as the hemifumarate salt (Formula la), an exemplary method comprising administration of Compound 1 as the hemifumarate in an amount of between 20 mg and 120 mg per day, for example between 20 mg and 80 mg per day. In some cases, the amount is between 30 mg and 100 mg per day, for example between 30 mg and 60 mg per day. In some cases, the amount is between 30 mg and 50 mg per day, for example about 40 mg per day. In some cases, the amount is between 60 mg and 100 mg per day, for example about 80 mg per day.

Lower doses have also been investigated and are envisaged. Accordingly, it will be appreciated that in any method described herein the method may comprise administration of Compound 1 in an amount of between 9 mg and 108 mg per day, for example between 9 mg and 72 mg per day. Where Compound 1 is administered as the hemifumarate salt, in any method described herein the method may comprise administration of Compound 1 as the hemifumarate salt in an amount of between 10 mg and 120 mg per day, for example between 10 mg and 80 mg per day.

Inhibition of the PI3Kδ pathway in patients can be demonstrated by measuring the pharmacodynamic activity (PD) of Compound 1 in blood samples. A specific PD marker for PI3Kδ inhibition in blood is CD63 on basophils. Treatment of patients with Compound 1 as the hemifumarate salt results in a dose dependent reduction of the percentage CD63 positive basophils in blood samples of patients comparable to that reported for other PI3Kδ inhibitors (for example idelalisib). Especially at the dose of 40 mg the percentage of CD63 positive basophils is low in all samples measured confirming effective inhibition of the PI3Kδ pathway during the course of treatment.

Accordingly, certain preferred embodiments relate to a dose of 40 mg of a salt of Formula la, or a dose of 36 mg of a compound of Formula I.

However, it will be appreciated that higher doses are encompassed and envisaged. For example, the dose may be 60 mg of a salt of Formula la, or a dose of 54 mg of a compound of Formula I. For example, the dose may be 80 mg of a salt of Formula la, or a dose of 72 mg of a compound of Formula I.

In some cases, lower doses are envisaged. For example the dose may be 10 mg of a salt of Formula la, or a dose of 9 mg of a compound of Formula I.

The inventors have found a once daily dose effective and well-tolerated. Once daily dosing offers advantages when compared to some known and used PI3K inhibitors (for example, idelasib is prescribed as a twice daily 150 mg dose unless dosage reduction due to adverse effects is required). A once daily dose improves patient experience and may improve patient compliance. Patients with, especially advanced, cancers often experience a considerable pill burden and may have difficulty swallowing.

Accordingly, in the methods of the invention the dose may be taken once daily. In other words, the dose is not divided and spaced throughout the day. It may be taken as in a single dosage unit (for example, a single tablet or capsule), or in multiple dosage forms (for example, as two or more tablets or capsules).

Accordingly, in some cases the method comprises administering one or more solid dosage units. For example, in some cases the daily dose is 40 mg and the method comprises administering two 20 mg solid dosage units (that is, a solid dosage unit comprising 20 mg of Compound 1 as the hemifumarate).

In another example, the daily dose may be 60 mg and administration may comprise three solid dosage units, each dosage unit comprising 20 mg of a salt of Formula la. In another example, the daily dose may be 80 mg and the administration may comprise four solid dosage units, each dosage unit comprising 20 mg of a salt of Formula la.

The inventors have identified that Compound 1 has a surprisingly good safety profile in humans, observing that treatment may result in less treatment-related grade 3 alanine aminotransferase (ALT) or aspartate aminotransferase (AST) elevation than would be expected for an inhibitor of this class. These results are described in GB2104416.9 (filed 29 March 2021).

Accordingly, in some cases treatment of patients with Compound 1 does not result in treatment-related grade 3 alanine aminotransferase (ALT) or aspartate aminotransferase (AST) elevation in more than 5% of patients. More preferably treatment of patients with Compound 1 does not result in treatment-related grade 3 alanine aminotransferase (ALT) or aspartate aminotransferase (AST) elevation in more than 1% of patients. Most preferably, treatment of patients with Compound 1 does not result in any clinically significant treatment-related alanine aminotransferase (ALT) or aspartate aminotransferase (AST) elevation in patients.

The inventors have further identified from initial trials that treatment with Compound 1 results in fewer adverse effects than is expected for inhibitors of this class. In particular, less common occurrence of serious diarrhoea and/or colitis is expected. These results are described in GB2104416.9 (filed 29 March 2021).

Accordingly, in some cases treatment of patients with Compound 1 does not result in treatment-related grade 3 diarrhoea or colitis in more than 5% of patients. More preferably treatment of patients with Compound 1 does not result in treatment-related grade 3 diarrhoea or colitis in more than 1% of patients. Most preferably, treatment of patients with Compound 1 does not result in any clinically significant treatment-related diarrhoea or colitis in patients.

The inventors have observed that treatment with Compound 1 does not appear to result in clinically significant treatment-related decrease in neutrophils below the normal range. These results are described in GB2104416.9 (filed 29 March 2021).

Accordingly, in some cases treatment of patients with Compound 1 does not result in treatment-related grade 3 neutropenia in more than 5% of patients. More preferably treatment of patients with Compound 1 does not result in treatment-related grade 3 neutropenia in more than 1% of patients. Most preferably, treatment of patients with Compound 1 does not result in any clinically significant treatment-related neutropenia in patients.

It will be appreciated that adverse effects may interrupt or even preclude ongoing treatment, depending on their severity. Where serious adverse effects are noted, treatment may be paused, dosage adjusted downwards (with potentially deleterious influence on efficacy) or even lead to a decision to terminate treatment. In the treatment of cancer, any of these interruptions may have significant negative effects on patient health, prognosis, and/or morale.

Its excellent safety profile means that Compound 1 offers treatment regimens that may be suitable for long term use without interruption. Accordingly, it is envisaged that patients may be prescribed a treatment regimen for a duration of months. In some cases, the treatment is prescribed for at least 1 month. In some cases, the treatment is prescribed for at least 2 months. In some cases, the treatment is prescribed for at least 3 months. In some cases, the treatment is prescribed for at least 4 months. In some cases, the treatment is prescribed for at least 5 months. In some cases, the treatment is prescribed for at least 6 months. In some cases, the treatment is prescribed for at least 1 year.

In some cases, the treatment duration is at least 1 month without interruption. In some cases, the treatment duration is at least 2 months without interruption. In some cases, the treatment duration is at least 3 months without interruption. In some cases, the treatment duration is at least 4 months without interruption. In some cases, the treatment duration is at least 5 months without interruption. In some cases, the treatment duration is at least 6 months without interruption. In some cases, the treatment duration is at least 1 year without interruption.

It will be appreciated that the methods of the invention may be particularly useful in the treatment of certain patient groups, for example elderly and/or frail patients who are not presently eligible to receive PI3Kδ inhibitors because of their poor tolerability.

Accordingly, in some cases the patient is 50 years old or older, for example 55 years old or older, for example 60 years old or older, for example 65 years old or older, for example 70 years old or older, for example 75 years old or older, for example 80 years old or older.

In some cases, the patient is or would be considered by a physician unsuitable for treatment with idelalisib and/or other PI3Kδ inhibitors.

In some cases, the patient is a patient who has been previously diagnosed as suffering from a gastrointestinal disorder, for example colitis or chronic diarrhoea.

### Combination with Compound 2

The inventors have observed that a combination of Compound 1 and Compound 2 shows apparent synergistic activity on high-grade and acinar-to-ductal metaplasia (ADM) tumour burden as well as high-grade and ADM number of lesions. The combination of Compound 1 and Compound 2 also shows apparent synergistic activity on pancreatic cancer associated activities including tumour fibrosis, tumour T regulatory cells (Tregs) and cancer associated fibroblasts (CAFs).

In some embodiments, the invention provides Compound 1, or a pharmaceutically acceptable salt thereof, for use in a method of treatment of pancreatic cancer, wherein the method comprises administration of Compound 1, or a pharmaceutically acceptable thereof, and Compound 2, or a pharmaceutically acceptable salt thereof.

In some embodiments, the invention provides a combination of Compound 1, or a pharmaceutically acceptable thereof, and Compound 2, or a pharmaceutically acceptable salt thereof, for use in a method of treating pancreatic cancer.

The dose of Compound 2 may be provided once daily (QD), preferably twice daily (BID), preferably but not necessarily administered orally. Other methods of administration may be used. A suitable daily dose may be between 5 mg and 2 g, for example between 10 mg and 1g.

Compound 1 and Compound 2 may be administered simultaneously or at different times. For example, Compound 1 may be administered once daily, and Compound 2 may be administered twice daily. Suitably, but not necessarily, Compound 1 and Compound 2 are provided in separate formulations (for example, separate dosage units such as tablets or capsules).

### Combination with a checkpoint inhibitor

The inventors have observed that a combination of Compound 1 and a checkpoint inhibitor may be advantageous, a combination of Compound 1 and an anti-PD-1 antibody showing much stronger anti-tumour growth activity compared to the anti-PD-1 antibody alone. Body weight data suggest the combination may be well-tolerated.

Accordingly, in some embodiments the invention provides Compound 1, or a pharmaceutically acceptable salt thereof, for use in a method of treatment of pancreatic cancer, wherein the method comprises administration of Compound 1, or a pharmaceutically acceptable thereof, and a checkpoint inhibitor. Suitably, the checkpoint inhibitor is a PD-1 or PD-L1 inhibitor. In some cases, it is a PD-1 inhibitor. In some cases, it is a PD-L1 inhibitor.

### Combination with a further chemotherapeutic agent

In some embodiments, a method described herein may comprise administration of a therapeutically effective amount of an additional chemotherapeutic agent, optionally two additional chemotherapeutic agents. Suitable chemotherapeutics agents include gemcitabine and nab-paclitaxel. Thus, methods of the present invention may comprise administration of gemcitabine and/or nab-paclitaxel.

Accordingly, in some cases the methods of the invention are directed to combination therapy, the combination therapy comprising treatment of a patient with Compound 1 or a pharmaceutically acceptable salt thereof and an additional chemotherapeutic agent, for example gemcitabine (Gemzar^{®}) or nab-paclitaxel (Abraxane^{®}). Optionally, a checkpoint inhibitor may also be administered.

It will be appreciated that Compound 1 and the additional chemotherapeutic agent(s) will suitably, although not necessarily, be given at different times and/or on different schedules and may be formulated for administration by different routes. For example, Compound 1 or a pharmaceutically acceptable salt thereof may be given as an oral dose, for example, a daily oral dose, while the additional chemotherapeutic agent may be given as infusion. For example both gemcitabine and nab-paclitaxel may be given in a 28 day cycle on days 1, 8, and 15.

In some cases, the combination therapy comprises treating the patient with Compound 1 or a pharmaceutically acceptable salt thereof and gemcitabine and nab-paclitaxel.

### Formulations

Compound 1 is suitably provided as a hemifumarate as described above. Suitability, Compound 1 is provided in a pharmaceutical composition formulated for oral administration. The pharmaceutical composition may be provided in a capsule or may be provided in a tablet. In some cases, it is provided in a tablet for example, as a coated or non-coated tablet produced by compression of a powdered or granulated composition. In other cases, it is provided in a capsule, for example, as a powdered or granulated composition within a hard- or soft-shell capsule, for example, a hydroxymethyl cellulose (HPMC) capsule. In other words, an oral dosage form is preferred.

The formulation suitably comprises one or more pharmaceutically acceptable fillers, disintegrants, glidants, and/or lubricants.

In some cases, the oral dosage form may comprise 5 mg of Compound 1, provided as the hemifumarate. In some cases, the oral dosage form may comprise 20 mg of Compound 1, provided as the hemifumarate. Both oral dosage forms have been made as described in Table 1.

It will however be appreciated that for some patients, smaller dosage forms may be preferred, for example if a patient has difficulties swallowing. Even using the smaller 5 mg solid dosage form, the burden on patients (number of capsules or tablets) is not uncommon in treatment of the conditions claimed.

**Table 1**

| **Ingredient** | **Function** | **Quantity per capsule (mg)** | |
|---|---|---|---|
| | | **5 mg** | **20 mg** |
| Compound 1 hemifumarate | Active substance | 5.00* | 20.00* |
| Microcrystalline cellulose | Filler | 24.34 | 97.35 |
| Mannitol | Filler | 24.34 | 97.35 |
| Croscarmellose sodium | Disintegrant | 2.26 | 9.04 |
| Silica, colloidal hydrated | Glidant | 0.28 | 1.13 |
| Magnesium stearate (vegetable origin) | Lubricant | 0.28 | 1.13 |
| Hydroxypropyl methyl cellulose (HPMC) capsule, size 5 (5x11mm), white | Capsule shell | 1 | NA |
| Hydroxypropyl methyl cellulose (HPMC) capsule, size 1 (6x19mm), white | Capsule shell | NA | 1 |
| **Total capsule fill weight** | | **56.50** | **226.00** |

| | | | |
|---|---|---|---|
| *Corrected for purity (according to Certificate of Analysis). | | | |

The oral dosage form may also comprise 40 mg of Compound 1, provided as the hemifumarate. The oral dosage form may also comprise 80 mg of Compound 1, provided as the hemifumarate. Both oral dosage forms can be made similarly to the dosage forms described **Table 1**. It will be appreciated that, for the doses of the method, the lowest dosage burden (number of capsules or tablets) is placed on patients, when a single solid dosage form is used. This is most advantageous for patient compliance.

Accordingly, the invention further relates to a pharmaceutical composition comprising Compound 1, preferably provided as the hemifumarate salt, formulated for oral administration.

An exemplary formulation includes Compound 1 hemifumarate, microcrystalline cellulose, mannitol, croscarmellose sodium and magnesium stearate. The formulation may be provided in a solid dosage form, for example as tablet or powder or granulated composition encapsulated in a shell capsule. It will be appreciated that a single tablet can accommodate a higher dose of Compound 1 hemifumarate than a single capsule. Therefore, tablets are preferred for higher dose units. Tablets may be coated to improve taste or swallowing.

The amount of Compound 1 hemifumarate may be 5 mg to 20 mg, for example 5 mg or 20 mg. In another embodiment the amount of compound 1 may be 5 mg to 80 mg, for example 5 mg, 20 mg, 40 mg or 80 mg.

Accordingly, in some embodiments Compound 1 is provided as a solid dosage unit comprising a pharmaceutical composition comprising 5 mg of Compound 1 hemifumarate. Accordingly, in some embodiments Compound 1 is provided as a solid dosage unit comprising a pharmaceutical composition comprising 20 mg of Compound 1 hemifumarate. Accordingly, in some embodiments the invention provides a solid dosage unit comprising a pharmaceutical composition comprising 40 mg of Compound 1 hemifumarate. Accordingly, in some embodiments the invention provides a solid dosage unit comprising a pharmaceutical composition comprising 80 mg of Compound 1 hemifumarate.

Accordingly, in some embodiments the invention provides a tablet comprising a pharmaceutical composition comprising 40 mg of Compound 1 hemifumarate. Accordingly, in some embodiments the invention provides a tablet comprising a pharmaceutical composition comprising 80 mg of Compound 1 hemifumarate. It will be appreciated that the pharmaceutical composition of the tablet can be similar to that of the capsule or can be optimised for tableting.

Suitability, Compound 2 is provided in a pharmaceutical composition formulated for oral administration. The pharmaceutical composition may be provided in a capsule or may be provided in a tablet. In some cases, it is provided in a tablet. In other cases, it is provided in a capsule, for example, as a powdered or granulated composition or a liquid composition within a hard- or soft-shell capsule, for example, a gelatin or hydroxymethyl cellulose (HPMC) capsule. In other words, an oral dosage form is preferred.

The formulations suitably comprise one or more pharmaceutically acceptable fillers, disintegrants, glidants, and/or lubricants.

Checkpoint inhibitors and/or additional chemotherapeutic agents may be provided in any suitable formulation and any suitable dosage, by any suitable means of administration. Suitably, but not necessarily, checkpoint inhibitors and/or additional chemotherapeutic agents may be approved drugs prescribed and administered according to the label.

### Treatment of pancreatic cancer using Compound 1

Compound 1 is a PI3Kδ inhibitor. It is recognised in the art that the PI3K pathway is frequently activated in pancreatic cancer. Accordingly, the methods of the present invention are directed to the treatment of pancreatic cancer, such as but not limited to pancreatic ductal adenocarcinoma (PDAC) and pancreatic neuroendocrine tumours (PanNETs or PNETs).

The methods of the present invention may therefore relate to treatment of pancreatic cancer characterised by upregulation of the PI3K pathway. The methods of the present invention may therefore relate to treatment of pancreatic cancer by modulation of PI3K pathway in a patient.

The pancreatic cancer may be any exocrine or neuroendocrine pancreatic cancer type. Accordingly, the methods of the present invention are directed to the treatment of pancreatic cancer, such as but not limited to pancreatic ductal adenocarcinoma (PDAC) and pancreatic neuroendocrine tumours (PanNETs or PNETs). In some cases, the pancreatic cancer is pancreatic ductal adenocarcinoma. In some cases, the pancreatic cancer is pancreatic neuroendocrine tumours.

The treatment may be a monotherapy or a combination therapy as described herein.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

### Examples

### EXAMPLE 1

### Activity of Compound 1 with or without an ATX inhibitor in the KPC mouse model of pancreatic cancer

The KPC mouse is an established and clinically relevant model of pancreatic ductal adenocarcinoma (PDAC) which develops many key features observed in human PDAC including pancreatic intraepithelial neoplasia alongside a robust inflammatory reaction including exclusion of effector T cells. Metastases are observed in around 80% of the mice and are located primarily in the liver and lungs. Mutations in both KRAS and TP53 genes are found in around 80% and 70% of all human PDACs respectively. The genetic background of KPC mouse contains *IoxP* sites flanking exons 2-10 of the transformation related protein 53 gene (p53^{floxflox}), and a conditional activation point mutation in the KRAS gene (KRAS G12D). A lox-stop-lox termination sequence is encoded upstream of KRAS mutated genes to prevent expression in the absence of Cre recombinase. Exposure to Cre recombinase removes the floxed p53 sequence - creating a null allele (+/-). The pancreas-specific Pdx-1 promoter enables expression of Cre recombinase in acini, islet and duct cells of the pancreas. Cre-mediated recombination excises the lox-stop-lox termination sequences and enables expression of KRAS G12D in pancreatic tissue. This genetic background leads to the development of malignant cells early after conception and results in tumour growth to which mice succumb when left untreated.

At 5 weeks of age, KPC mice were treated with either Compound 1 in drinking water, Compound 2 (an ATX inhibitor) in mixed chow, or a combination of the two drugs. Control animals received normal drinking water and standard chow. Six weeks after initiation of treatment, i.e. at 11 weeks of age, treatment was stopped and mice were euthanized. Pancreata were harvested and analysed by histological analysis for number of lesions and tumour burden by arbitrary scoring units for grade of differentiation (from acinar-to-ductal metaplasia [ADM] to increasing severity of pancreatic intraepithelial neoplasia [PanIN-1/2/3] and PDAC).

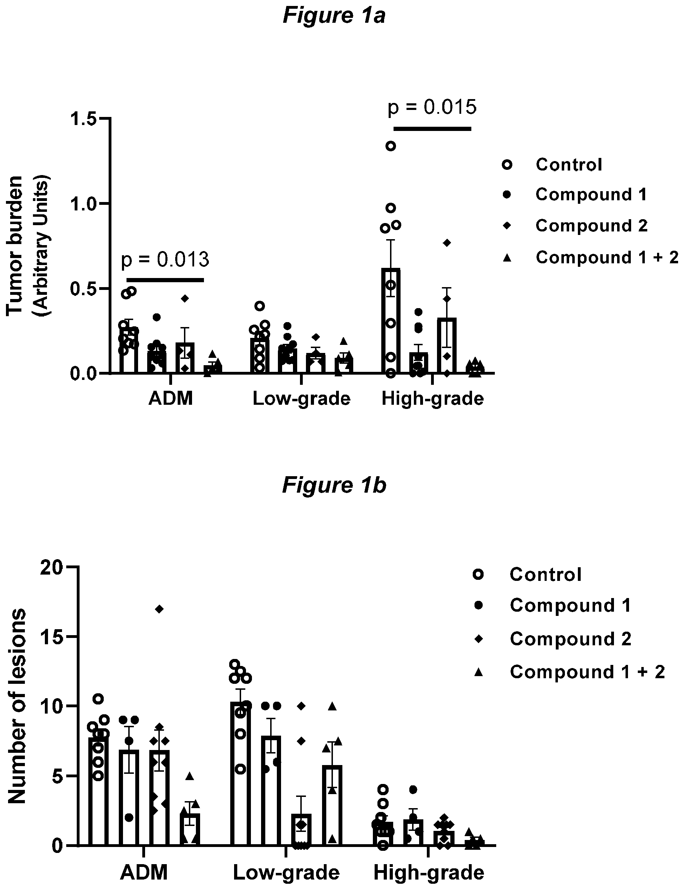

As shown in **Figure** 1, Compound 1 alone reduces high-grade (PanIN-3 and PDAC) tumour burden without having significant effects on the number of lesions in this model. Compound 2 alone in the model reduces the number of low-grade (PanIN-1 and PanIN-2) lesions without having significant effects tumour burden. Surprisingly, the combination of Compound 1 and Compound 2 shows an apparent synergistic activity on high-grade and ADM tumour burden as well as high-grade and ADM number of lesions.

### EXAMPLE 2

### Activity of Compound 1 in combination with a checkpoint inhibitor in the syngeneic Pan02 mouse model of pancreatic cancer (anti-mouse PD-1 mAb clone RMP1-14)

Pan02 is a well-established pancreatic ductal adenocarcinoma (PDAC) mouse model and originates from tumour cells that were chemically induced (3-methylcholanthrene, 3-MCA) in male C57BL/6 mice. The non-immunogenic ('cold') tumour profile and relatively slow tumour growth of this model makes this an ideal model for the evaluation of anti-cancer immunotherapy responses and pharmacodynamics of the immune system.

Female 6-8 week old C57BL/6 mice, 10 per group, were subcutaneously injected with 3 x 10⁶ cells in 100µl serum free RPMI medium into the flank. When the average tumour volume reached 60 mm³, animals were randomly allocated to the different study groups: vehicle, anti-PD-1 alone and anti-PD-1 with Compound 1. Vehicle (1% methylcellulose solution), Compound 1 (30 mg/kg) was dosed twice daily (BID) via oral gavage. Anti-PD1 (10 mg/kg) in PBS was dosed twice weekly (BIW) via intraperitoneal (IP) injection.

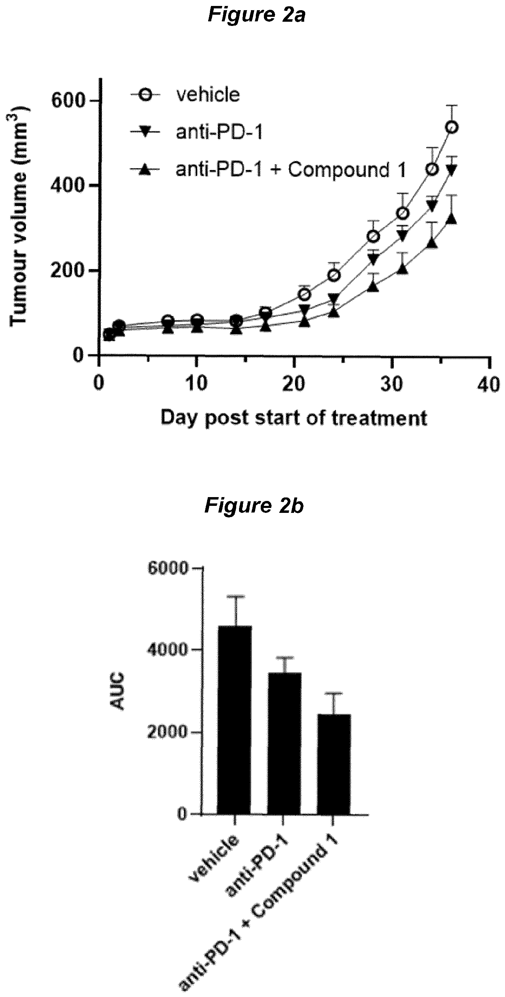

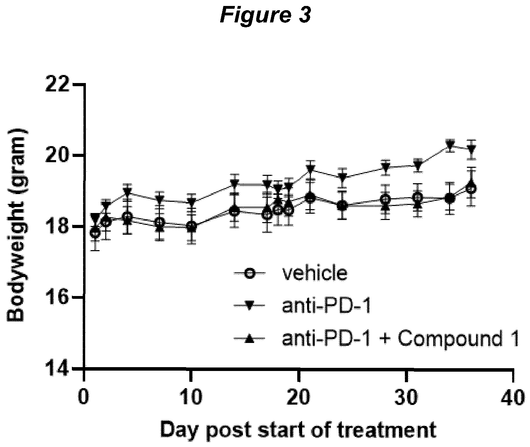

As seen in **Figure 2** from the tumour volume and area under the curve (AUC), the anti-PD-1 antibody has only a modest effect on tumour outgrowth in this model. Surprisingly, the combination of the anti-PD-1 antibody with Compound 1 shows much stronger anti-tumour growth activity compared to the anti-PD-1 antibody alone. Furthermore, as also can be observed in **Figure 3**, Compound 1 and the combination of the anti-PD-1 antibody with Compound 1 do not result in any body weight loss and are thus well tolerated. This is surprising given the poor tolerability observed for idelalisib in pancreatic cancer patients and the warning letter issued by the US Food and Drug Administration (FDA) on the increased rate of adverse events, including deaths, in clinical trials with idelalisib in combination with other cancer medicines (Borazanci et al 2020).

### EXAMPLE 3

### Activity of Compound 1 with or without an ATX inhibitor in the KPC/WCB3 orthotopic mouse model of pancreatic cancer

The therapeutic efficacy of Compound 1 with or without ATX inhibitor was evaluated in the orthotopic KPC/WCB3 mouse model of pancreatic cancer. This model is constituted by the implantation of KPC/WBC3 cells originating from a spontaneous developed donor tumour in KPC mice into the pancreas of male wildtype C57BL/6J recipient mice. The pathology and tumour microenvironment in this model closely resemble human pancreatic cancer.

2 x 10⁵ KPC/WCB3 cells were orthotopically injected into the pancreas of 8-week-old mice (Charles River Laboratories). Treatments with Compound 1 in drinking water, Compound 2 in mixed chow, or a combination of the two drugs were started 7 days after the injection of KPC/WCB3 cells. Control animals received normal drinking water and standard chow. One month after initiation treatment was stopped and mice were euthanized. Pancreata were harvested and analysed by histological analysis for level of fibrosis, number of Tregs and number of proliferating CAFs.

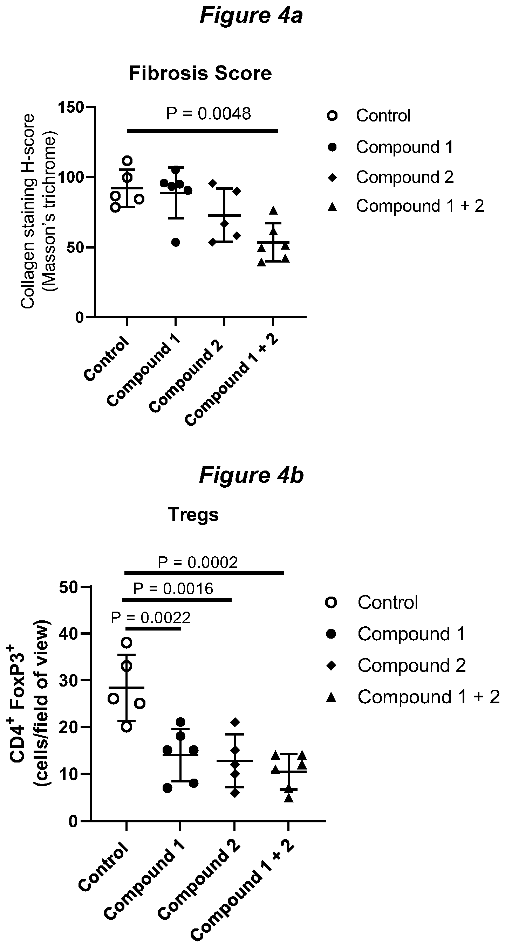

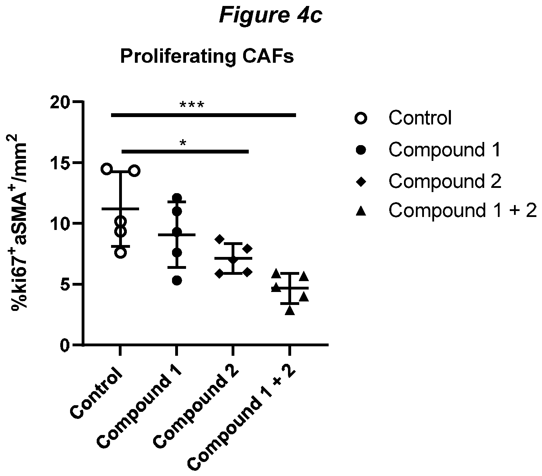

As shown in **Figure** 4, Compound 1 alone has a limited effect on tumour fibrosis score and proliferating CAFs but significantly reduces Tregs. Compound 2 alone has a limited effect on tumour fibrosis score but significantly reduces Tregs as well as proliferating CAFs. Surprisingly, the combination of Compound 1 and Compound 2 shows an apparent synergistic activity on fibrosis score as well as proliferating CAFs whilst maintaining a strong reduction of Tregs.

### References

A number of publications are cited above in order to more fully describe and disclose the invention and the state of the art to which the invention pertains. Full citations for these references are provided below.
WO2011058149
Borazanci, E., Pishvaian, M. J., Weekes, C., Huang, J. & Rajakumaraswamy, N. A Phase lb Study of Single Agent Idelalisib Followed by Idelalisib in Combination with Chemotherapy in Patients with Metastatic Pancreatic Ductal Adenocarcinoma. Oncol (2020) doi:10.1634/theoncologist.2020-0321.
Burris, H. A. et al. Improvements in survival and clinical benefit with gemcitabine as first-line therapy for patients with advanced pancreas cancer: a randomized trial. J Clin Oncol 15, 2403-2413 (1997).
Conway, J. R., Herrmann, D., Evans, T. J., Morton, J. P. & Timpson, P. Combating pancreatic cancer with PI3K pathway inhibitors in the era of personalised medicine. Gut 68, 742 (2019).
Haselmayer, P. et al., Frontiers in Immunology (2014), Vol. 5, Art. 233, p. 1-15; see p. 2, col. 2, section "Chemical Synthesis", par. 1
Macqueen et al. (2020), "Abstract 666: A novel highly selective PI3K[delta] inhibitor for the treatment of solid malignancies that express high levels of target protein as assessed by immunochemistry", CANCER RESEARCH, vol. 80, no 16 Supplement, 15 August 2020, page 666, XP055941409.
Papakonstanti et al. (2019) "Preclinical development of a novel, highly selective PI3K[delta] inhibitor, IOA-244 for the treatment of solid malignancies", 7 November 2019, page 1, XP055941402, London, UK.
Johnson et al (2019) - "Preclinical development of a novel, highly selective PI3Kdelta inhibitor, IOA- 244, for the treatment of solid malignancies", 9 November 2019, XP055941748.
For standard molecular biology techniques, see Sambrook, J., Russel, D.W. Molecular Cloning, A Laboratory Manual. 3 ed. 2001, Cold Spring Harbor, New York: Cold Spring Harbor Laboratory Press.

## Claims

1. A compound of Formula I: 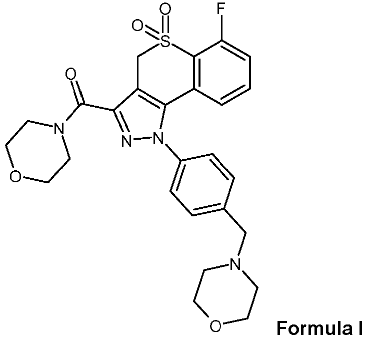 or a pharmaceutically acceptable salt thereof for use in a method of treatment of pancreatic cancer in a patient.

2. The compound or salt for use according to claim 1, wherein the method further comprises administration of a compound of Formula II: 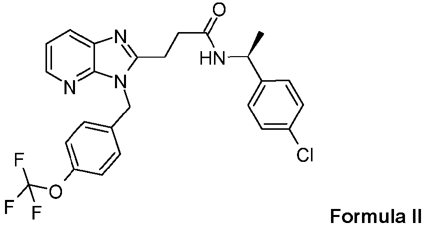 or a pharmaceutically acceptable salt thereof.

3. The compound or salt for use according to claim 1 or claim 2, wherein the compound of Formula I is administered in a pharmaceutical composition comprising said compound or a pharmaceutically acceptable salt thereof and a pharmaceutically acceptable diluent, carrier or excipient; wherein the pharmaceutical composition is suitable for oral administration.

4. The compound or salt for use according to claim 2 or claim 3, wherein the compound of Formula II is administered in a pharmaceutical composition comprising said compound or a pharmaceutically acceptable salt thereof and a pharmaceutically acceptable diluent, carrier or excipient; wherein the pharmaceutical composition is suitable for oral administration.

5. The compound or salt for use according to any preceding claim, wherein the method further comprises administration of a checkpoint inhibitor.

6. The compound or salt for use according to claim 5, wherein the checkpoint inhibitor is an anti-PD-1 and/or anti-PDL1 monoclonal antibody.

7. The compound or salt for use according to any preceding claim, wherein the method comprises administration of a therapeutically effective amount of an additional chemotherapeutic agent.

8. The compound or salt for use according to claim 7, wherein the additional chemotherapeutic agent is gemcitabine.

9. The compound or salt for use according to claim 7, wherein the additional chemotherapeutic agent is nab-paclitaxel.

10. The compound or salt for use according to claim 7, wherein two additional chemotherapeutic agents are used.

11. The compound or salt for use according to claim 10, wherein the two additional chemotherapeutic agents are gemcitabine and nab-paclitaxel.

12. The compound or salt for use according to any preceding claim, wherein the pancreatic cancer is pancreatic ductal adenocarcinoma (PDAC).

## Patentansprüche

1. Verbindung der Formel I: 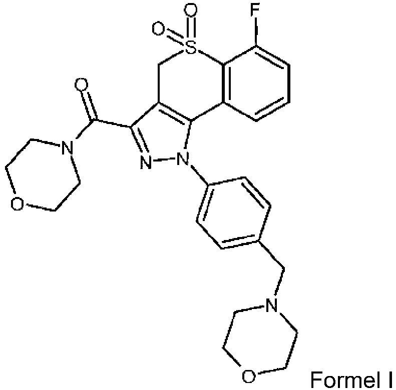 oder ein pharmazeutisch annehmbares Salz davon zur Verwendung in einem Verfahren zur Behandlung von Bauchspeicheldrüsenkrebs bei einem Patienten.

2. Verbindung oder Salz zur Verwendung nach Anspruch 1, wobei das Verfahren weiters die Verabreichung einer Verbindung der Formel II: 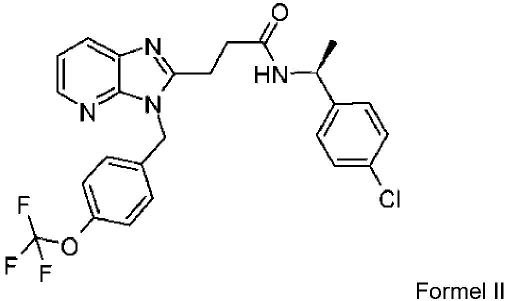 oder eines pharmazeutisch annehmbaren Salzes davon umfasst.

3. Verbindung oder Salz zur Verwendung nach Anspruch 1 oder Anspruch 2, wobei die Verbindung der Formel I in einer pharmazeutischen Zusammensetzung verabreicht wird, welche die Verbindung oder ein pharmazeutisch annehmbares Salz davon und einen pharmazeutisch annehmbaren Verdünner, Träger oder Hilfsstoff umfasst; wobei die pharmazeutische Zusammensetzung zur oralen Verabreichung geeignet ist.

4. Verbindung oder Salz zur Verwendung nach Anspruch 2 oder Anspruch 3, wobei die Verbindung der Formel II in einer pharmazeutischen Zusammensetzung verabreicht wird, welche die Verbindung oder ein pharmazeutisch annehmbares Salz davon und einen pharmazeutisch annehmbaren Verdünner, Träger oder Hilfsstoff umfasst; wobei die pharmazeutische Zusammensetzung zur oralen Verabreichung geeignet ist.

5. Verbindung oder Salz zur Verwendung nach einem der vorangegangenen Ansprüche, wobei das Verfahren weiters die Verabreichung eines Checkpoint-Inhibitors umfasst.

6. Verbindung oder Salz zur Verwendung nach Anspruch 5, wobei der Checkpoint-Inhibitor ein monoklonaler Anti-PD-1- und/oder Anti-PDL1-Antikörper ist.

7. Verbindung oder Salz zur Verwendung nach einem der vorangegangenen Ansprüche, wobei das Verfahren die Verabreichung einer therapeutisch wirksamen Menge eines weiteren Chemotherapeutikums umfasst.

8. Verbindung oder Salz zur Verwendung nach Anspruch 7, wobei das weitere Chemotherapeutikum Gemcitabin ist.

9. Verbindung oder Salz zur Verwendung nach Anspruch 7, wobei das weitere Chemotherapeutikum Nab-Paclitaxel ist.

10. Verbindung oder Salz zur Verwendung nach Anspruch 7, wobei zwei weitere Chemotherapeutika verwendet werden.

11. Verbindung oder Salz zur Verwendung nach Anspruch 10, wobei die zwei weiteren Chemotherapeutika Gemcitabin und Nab-Paclitaxel sind.

12. Verbindung oder Salz zur Verwendung nach einem der vorangegangenen Ansprüche, wobei der Bauchspeicheldrüsenkrebs duktales Adenokarzinom des Pankreas (PDAC) ist.

## Revendications

1. Composé de formule I : 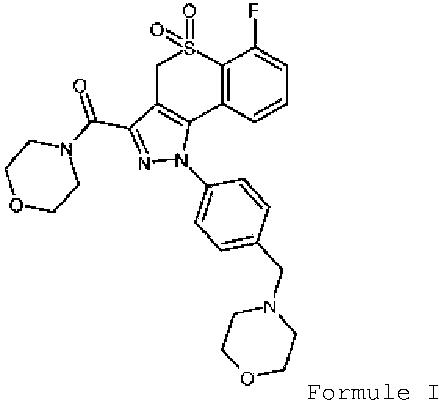 ou sel pharmaceutiquement acceptable de celui-ci pour utilisation dans une méthode de traitement du cancer du pancréas chez un patient.

2. Composé ou sel pour utilisation selon la revendication 1, dans lequel la méthode comprend en outre l'administration d'un composé de formule II : 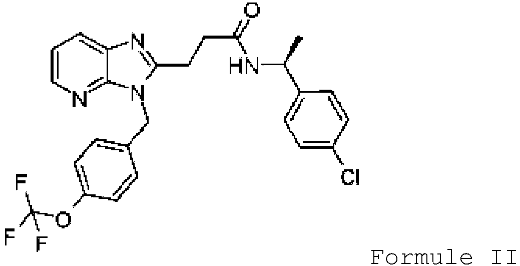 ou d'un sel pharmaceutiquement acceptable de celui-ci.

3. Composé ou sel pour utilisation selon la revendication 1 ou la revendication 2, dans lequel le composé de formule I est administré dans une composition pharmaceutique comprenant ledit composé ou un sel pharmaceutiquement acceptable de celui-ci et un diluant, un support ou un excipient pharmaceutiquement acceptable ; dans lequel la composition pharmaceutique est appropriée pour une administration orale.

4. Composé ou sel pour utilisation selon la revendication 2 ou la revendication 3, dans lequel le composé de formule II est administré dans une composition pharmaceutique comprenant ledit composé ou un sel pharmaceutiquement acceptable de celui-ci et un diluant, un support ou un excipient pharmaceutiquement acceptable ; dans lequel la composition pharmaceutique est appropriée pour une administration orale.

5. Composé ou sel pour utilisation selon une quelconque revendication précédente, dans lequel le procédé comprend en outre l'administration d'un inhibiteur de point de contrôle.

6. Composé ou sel pour utilisation selon la revendication 5, dans lequel l'inhibiteur de point de contrôle est un anticorps monoclonal anti-PD-1 et/ou anti-PDL1.

7. Composé ou sel pour utilisation selon une quelconque revendication précédente, dans lequel le procédé comprend l'administration d'une quantité thérapeutiquement efficace d'un agent chimiothérapeutique supplémentaire.

8. Composé ou sel pour utilisation selon la revendication 7, dans lequel l'agent chimiothérapeutique supplémentaire est la gemcitabine.

9. Composé ou sel pour utilisation selon la revendication 7, dans lequel l'agent chimiothérapeutique supplémentaire est le nab-paclitaxel.

10. Composé ou sel pour utilisation selon la revendication 7, dans lequel deux agents chimiothérapeutiques supplémentaires sont utilisés.

11. Composé ou sel pour utilisation selon la revendication 10, dans lequel les deux agents chimiothérapeutiques supplémentaires sont la gemcitabine et le nab-paclitaxel.

12. Composé ou sel pour utilisation selon une quelconque revendication précédente, dans lequel le cancer du pancréas est un adénocarcinome canalaire pancréatique (PDAC).
